Europäisches Patentamt

European Patent Office

Office européen des brevets

(11) Publication number: **0 404 317**
**A2**

# EUROPEAN PATENT APPLICATION

(21) Application number: **90304837.9**

(22) Date of filing: **03.05.90**

(51) Int. Cl.⁵: **C12N 1/00, C11D 1/722**

(30) Priority: **22.06.89 US 370415**

(43) Date of publication of application:
**27.12.90 Bulletin 90/52**

(84) Designated Contracting States:
**DE ES FR GB IT NL**

(71) Applicant: **PETROLITE CORPORATION**
**369 Marshall Avenue**
**Saint Louis Missouri 63119(US)**

(72) Inventor: **McCarthy, Kevin J.**
**7360 Teasdale**
**University City, Missouri 63130(US)**
Inventor: **Burkhardt, Charles W.**
**1415 New Smizer Mill Road**
**Fenton, Missouri 63026(US)**
Inventor: **Stehlin, Mark P.**
**9517 Eucalyptus**
**St. Louis, Missouri 63123(US)**

(74) Representative: **Seaborn, George Stephen et al**
**c/o Edward Evans & Co. Chancery House**
**53-64 Chancery Lane**
**London WC2A 1SD(GB)**

(54) **Use of ethoxylated propoxylated alcohols to prevent or reduce foam in fermentation broths.**

(57) Compounds of the formula
$RO(EtO)_n(PrO)_mH$
are effective as antifoamers and defoamers in (pharmaceutical) fermentation broths.

EP 0 404 317 A2

# USE OF ETHOXYLATED PROPOXYLATED ALCOHOLS TO PREVENT OR REDUCE FOAM IN FERMENTATION BROTHS

## Background of the Invention

This invention relates to a method for the prevention and reduction of foam in fermentation broths. More particularly, it relates to such a method wherein the foam is controlled by the addition of chemical agents.

Foams occur as an undesirable incidental feature in many systems. Although some theories of foam formation have been proposed, little is known about why some compounds are antagonistic to foams while others are not. Of considerable frustration to those in the art of foam control is the fact that each foam control problem is unique, calling for a different compound for effective foam control. Some materials are useful only to prevent foam formation ("antifoamers") while some are useful only to reduce foam which has already formed ("defoamers"). Efforts to predict which compound will work in a given system have not proven successful. As a result, foam control is basically an empirical art.

The following quotations summarize the views of experts in the field of foam control:

"More than 300,000 chemicals are now available, thousands of which work as defoamer ingredients in particular systems. It is difficult then to pinpoint exactly which defoamer will work where, and why, so it is best to leave the problem to specialists. Another reason is that all variables must be considered when formulating defoamers compatible with production systems."

Foam Control, A Misunderstood Concept, by Marshall Ott, Household & Personal Products Industry, Feb. 1978.

"Because of the variety of materials to be defoamed and the extreme conditions of operation, no single material has universal application as a chemical antifoam. Some systems are predominantly aqueous, whereas others are organic mixtures with only a trace of moisture; in others the presence or absence of solids may be a factor. Operational conditions vary from high pressure steam generation to vacuum distillation; jet fuels may foam on the plane's ascent, and low-boiling substances may cause foaming in the oil-well pumping of certain gaseous petroleum crudes. To add to the difficulties, the antifoam for one system may be a foamer in another." (Emphasis added).

Encyclopedia of Polymer Science and Technology, Vol. 2, 1964, pg. 164 (Antifoaming Agents).

"Thus a paper mill may easily use six or more defoamers for routine coated paper manufacture. The same holds true for other defoamer applications. For example, a paint manufacturer may need different defoamers for the various phases of paint production such as in the pigment grind, letdown, can filling, and mill effluent. Manufacturers of textiles, sugar from sugar beets, and fertilizers require different defoamers for the various phases of production."

Encyclopedia of Chemical Technology, Vol. 7, 1979, pg. 433 (Defoamers).

"One of the fruits of success in this endeavor would be to diminish the empiricism that at present characterizes the search for an optimum foam inhibitor."

Mechanisms of Foam Stabilization and Antifoaming Action, by S. Ross, Rensselaer Polytechnic Institute, Chemical Engineering Progress. Vol. 63, #9, pg. 46.

"It is well known that the performance of an antifoam or foam preventer is strongly dependent on the type of foaming system involved. However, little is known about the specific parameters which influence antifoaming activity."

Mechanism of Antifoaming Action, by R.D. Kulkarni et al, J. of Colloid and Interface Science, Vol. 59, #3, May 1977.

"An important problem of commercial aerobic fermentation processes consists of the foaming of media, and the suppression of this tendency. In the fermentation of antibiotics it is particularly important to choose the correct method for the suppression of foaming, and to choose a suitable antifoam agent."

The Foams of Fermentation Broths, by Laszlo Szarka, Biotechnology & Bioengineering , Vol. XI, pg. 701, 1969.

Foaming in fermentation broths is particularly problematic in that the material chosen must be compatible with sterilization procedures, non-toxic to the fermentation organism, non-interfering with extract of the desired fermentation product, as well as be an effective foam control agent. In particular, the pharmaceutical industry has a long-felt need for more effective foam control agents.

From a technical standpoint there are a large number of instances where silicone foam control agents are preferred because of their superior performance. However, since silicones are often 3 to 10 times more expensive than other foam control compounds, the non-silicones are economically preferred, even if their performance is inferior. Therefore, it would be desirable to provide a highly effective and economical foam control agent suitable for use in fermentation broths.

Suppression of foams in fermentation broths is known. Examples of typically used compounds include silicone emulsions, silica dispersions, pure silicone fluids, polypropylene glycol, and various block copolymers of propylene oxide and ethylene oxide.

US 3,862,243 (Bellos - Petrolite) discloses compounds of the general formula

$$RO(EtO)_n(BuO)_mH$$

where R is a hydrocarbon group, EtO is an ethoxy moiety, BuO is a butoxy moiety, n is from 3 to 30 and m is from 2 to 20. This patent teaches that these compounds "are useful in controlling foams in many different types of systems. They control foam encountered in gas-treating systems in which a mixture of glycols and alkanolamines is used to dehydrate and purify natural gas; in activated-sludge-process sewerage plants, particularly in aeration basis and elsewhere; in protein adhesives solutions, such as casein and soybean adhesives as used in the plywood industry; in latex adhesives, printing inks; aqueous emulsions paints; etc."

Similar to the teachings of the above patent, commercial products are available which have the general formula

$$RO(EtO)_n(PrO)_mH$$

wherein PrO is a propoxy moiety. These compounds are sold as antifoamers for detergent systems such as dishwashing detergents, rinse aids, and metal spray cleaning detergent compounds; caustic scrubbers; and rolling oil formulations.

Numerous other polyalkoxylated compounds are also known and many of these compounds are known to be useful as foam control agents.

Summary of the Invention

The invention centers around the discovery that compounds of the formula

$$RO(EtO)_n(PrO)_mH$$

can be added to fermentation broths to prevent the formation of foam or to reduce foam which has already formed. The method of the invention is economical and surprisingly effective.

Detailed Description of the Invention

In the specification and claims, numerical values are not critical unless otherwise stated. That is, the numerical values may be read as if they were prefaced with the word "about" or "substantially".

The invention requires the use of a compound of the formula

$$RO(EtO)_n(PrO)_mH$$

wherein R is a $C_4$ to $C_{30}$ hydrocarbon radical, EtO is an ethoxy moiety, PrO is a propoxy moiety, n is from 10 to 40 and m is from 20 to 80.

R is defined as a "hydrocarbon radical", and in preferred embodiments has only carbon and hydrogen. However, in less preferred embodiments R can have minor substitutions, provided that such substitution does not substantially interfere with the desired foam control activity of the molecule, and thus in its broadest sense may be more precisely called a "substituted hydrocarbon radical". R is desirably an unsubstituted hydrocarbon group such as alkyl, alkenyl, aryl, cycloalkyl, alkaryl, etc., and more desirably is alkyl. Of alkyl groups, R is preferably straight chained. R should have 4 to 30, desirably 6 to 22, more desirably 8 to 18, preferably 12 to 16, and most preferably 14 carbon atoms.

EtO is an ethoxy moiety, preferably derived from ethylene oxide. The EtO moiety is present n times, n being 10 to 40, desirably 10 to 30, and preferably 15 to 25.

PrO is a propoxy moiety, preferably derived from propylene oxide. The PrO moiety is present m times, m being 20 to 80, desirably 25 to 60, preferably 30 to 50, and more preferably 35 to 45.

3

The compounds used in the invention are conveniently prepared by conventional processes well known to those skilled in the art. For instance, the alcohol corresponding to R is combined with a catalytic amount of an alkali metal hydroxide, heated, ethylene oxide is added and allowed to react, and propylene oxide is thereafter added and allowed to react.

The compounds are used in a fermentation broth. By "fermentation broth" is meant an aqueous dispersion of individual cells and nutrients for those cells, from which a metabolic product or cell culture is ultimately extracted; or an aqueous dispersion of the nutrients alone, prior to addition of the cells. Although the cells can be from multicellular organisms (particularly those from "immortal" cell lines or those used to support virus cultures), the cells are preferably bacteria or fungi but preferably not yeast. The desired metabolic product or cell culture extracted from the broth can be a cell culture but is preferably a metabolic product, more preferably a pharmaceutical. By "pharmaceutical" is meant a substance (other than a food) intended to be administered to a mammal to diagnose, cure, prevent, or treat a disease, by chemical action. Exemplary pharmaceuticals include antibiotics, hormones, steroids, and agents intended to alter immune response. Preferred pharmaceuticals are antibiotics.

In addition to water and optionally the cell line, the fermentation broth will contain nutrients for the cells. In general, the nutrients will comprise a source of carbon, nitrogen, trace elements (salts), and organism-specific requirements. Carbon sources include monosaccharides, disaccharides, polysaccharides, alcohols, carboxylic acids, fats, and hydrocarbons. Nitrogen sources often also include carbon, and are represented by ammonia, urea, bean meal, grain meal, seed meal, fish meal, cornsteep liquor, and yeast extracts. Trace elements can be specifically added (usually in the form of salts) but are often supplied by using well water or municipal tap water. Some organisms also require one or more specific additional compounds which they are not able to synthesize. These typically include amino acids, purines, or pyrimidines. The nutrients in fermentation broths are well known to those skilled in the art.

The oxyalkylated compounds useful in the invention are added to fermentation broths to prevent foam formation ("antifoamers") or to reduce foam which has already formed ("defoamers"). As an antifoamer the compounds are simply blended with the broth and as a defoamer the compounds are simply dropped or sprayed onto the foam. The use of diluents is not necessary and in fact is not desirable because of the possibility of the diluent interfering with either the fermentation process of the activity of the defoamer/antifoamer compounds.

As antifoamers the polyalkoxylated compounds are added in an effective amount. That is, an amount sufficient to retard the generation of foam in the broth. Although the precise amount will vary depending on the exact nature of the broth and the choice of polyalkoxylated compound, generally the polyalkoxylated compound will be used at 5 to 5000, desirably 25 to 3000, preferably 25 to 2000, and more preferably 50 to 1000 ppm (parts per million)(weight basis).

As defoamers the polyalkoxylated compounds are added in an effective amount. That is, an amount sufficient to reduce the volume of foam present on the broth. Since the precise amount will vary depending not only on the exact nature of the broth and the choice of polyalkoxylated compound, but also on the existing foam volume and the presence of any antifoam compounds, it is difficult to state in narrow terms the amount of polyalkoxylated compound to use. However, in general, the polyalkoxylated compound will be used at 5 to 5000, desirably 25 to 3000, preferably 25 to 2000, and more preferably 50 to 1000 ppm (parts per million)(weight basis).

The invention will be further illustrated in the following examples. In the examples, all parts and percentages are by weight unless otherwise specified. All footnotes are located at the end of the examples.

Example 1

A proprietary but commercial fermentation broth (used for antibiotic production) was supplied by a third party for evaluation. The broth had been sterilized, but may have been contaminated during sampling. The broth was not intentionally inoculated.

Antifoamer activity was evaluated by the following procedures:

1. All apparatus was thoroughly cleaned to remove any trace of chemicals from prior experiments.

2. 100 ml of the broth at room temperature was added to a 500 ml graduated cylinder.

3. An air pump was connected to a flow meter which was connected to a Pyrex® brand "12c" gas dispersion tube (ASTM 40-60, maximum pore diameter 25-50μm), and the air flow set at 2000 ml/min. The dispersion tube was placed in the broth at the bottom of the graduated cylinder.

4. The foam height was measured at 0.5, 1, 3, and 5 minutes. Since the graduated cylinder was only 500 ml, foam measurements in excess of 400 ml could not be made.

5. The procedure was then repeated with the addition (with gentle mixing) of various compounds (followed by the cleaning in Step 1). The results are shown in Table I. In the tables, the designation, for example, "14-30E-50P" means a $C_{14}$ n-alcohol (14) onto which has been reacted 30 moles of ethylene oxide (30E) and then 50 moles of propylene oxide (50P).

TABLE I

| Run | Defoamer | Diluent | Defoamer Dose (ppm) | Foam Height at X min (ml) | | | |
|-----|----------|---------|---------------------|---------|-------|-------|-------|
| | | | | 0.5 min | 1 min | 3 min | 5 min |
| 1* | None | | 0 | 140 | 160 | 200 | 215 |
| 2* | PPG-2000[1] | | 200 | 15 | 20 | 65 | 135 |
| 3* | PPG-2000[1] | | 200 | 15 | 25 | 75 | 125 |
| 4* | PPG-2000[1] | | 300 | 15 | 15 | 35 | 85 |
| 5* | PPG-2000[1] | | 500 | 15 | 10 | 15 | 30 |
| 6* | PPG-2000[1] | | 500 | 5 | 5 | 15 | 35 |
| 7 | 14-25E-50P | | 30 | 0 | 0 | 25 | 60 |
| 8 | 14-25E-50P | | 50 | 0 | 0 | 5 | 5 |
| 9 | 14-25E-50P | | 100 | 0 | 0 | 0 | 5 |
| 10 | 14-25E-50P | | 200 | 0 | 0 | 0 | 0 |

*Not an example of the invention.

## Example 2

The broth of Example I was aged for one day at (35°C) and then tested again. The results are shown in Table II.

TABLE II

| Run | Defoamer | Diluent | Defoamer Dose (ppm) | Foam Height at X min (ml) | | | |
|-----|----------|---------|---------------------|---------|-------|-------|-------|
| | | | | 0.5 min | 1 min | 3 min | 5 min |
| 1* | None | | 0 | 215 | 225 | 225 | 230 |
| 2* | PPG-2000[1] | | 200 | 35 | 40 | 115 | 190 |
| 3* | PPG-2000[1] | | 500 | 5 | 15 | 50 | 115 |
| 4* | PPG-2000[1] | | 800 | 0 | 5 | 35 | 75 |
| 5 | 14-25E-50P | | 50 | 0 | 0 | 15 | 50 |
| 6 | 14-25E-50P | | 100 | 0 | 0 | 5 | 15 |
| 7 | 14-25E-50P | | 200 | 0 | 0 | 0 | 0 |
| 8 | 14-25E-50P | | 1000 | 0 | 5 | 15 | 15 |
| 9 | 14-25E-50P | | 3000 | 35 | 40 | 115 | 140 |

*Not an example of the invention.

## Example 3

26 liters of the broth of Example 2 was treated with 200 ppm of the 14-25E-50P product and sterilized at 120°C for 5 minutes and then cooled. Foam which was present before the addition of the compound disappeared, and no new foam formed.

Example 4

A broth similar to the one in Example 1, but having worse foaming problems, was evaluated as in Example 1. The results are shown in Table III.

TABLE III

| Run | Defoamer | Diluent | Defoamer Dose (ppm) | Foam Height at X min (ml) | | | |
|-----|----------|---------|---------------------|-------------|-------|------------|-------|
| | | | | 0.5 min | 1 min | 3 min | 5 min |
| 1* | None | | 0 | 290 | 315 | 355 (2 min) | - |
| 2* | PPG-2000[1] | | 1000 | 40 | 60 | 290 | 340 |
| 3* | PPG-2000[1] | | 2000 | 15 | 30 | 85 | 285 |
| 4* | PPG-2000[1] | | 3000 | 10 | 15 | 130 | 275 |
| 5* | PPG-2000[1] | | 4000 | 5 | 20 | 90 | 240 |
| 6 | 14-25E-50P | | 300 | 65 | 100 | 130 | 105 |
| 7 | 14-25E-50P | | 500 | 5 | 15 | 25 | 35 |
| 8 | 14-25E-50P | | 700 | 10 | 15 | 20 | 20 |

*Not an example of the invention.

Example 5

In a manner similar to Example 2, the broth of Example 3 was aged for one day at 27°C and retested. The results are shown in Table IV.

TABLE IV

| Run | Defoamer | Diluent | Defoamer Dose (ppm) | Foam Height at X min (ml) | | | |
|-----|----------|---------|---------------------|-------------|-------|-------|-------|
| | | | | 0.5 min | 1 min | 3 min | 5 min |
| 1* | None | | 0 | +365 | - | - | - |
| 2* | PPG-2000[1] | | 250 | 115 | 165 | 265 | - |
| 3* | PPG-2000[1] | | 500 | 70 | 165 | 265 | - |
| 4* | PPG-2000[1] | | 1000 | 30 | 55 | 265 | 305 |
| 5 | 14-25E-50P | | 500 | 5 | 5 | 0 | 0 |

*Not an example of the invention.

Example 6

A proprietary, commercial fermentation broth having 7% undissolved solids (used for antibiotic production) was treated in a manner similar to Example 1, except that the fermentation broth was heated to 30°C and the air flow was 5.5 SCFH (2600 ml/min.) The results are shown in Table V.

TABLE V

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | >400 | - | - | - | - |
| 2 | 14-25E-50P | | 50 | 45 | 35 | 30 | 30 | 30 |
| 3 | 14-25E-50P | | 50 | 10 | 10 | 10 | 15 | 30 |
| 4 | 14-25E-50P | 2 | 100 | 35 | 30 | 35 | 45 | 75 |
| 5 | 14-30E-50P | | 50 | 50 | 15 | 40 | 50 | 125 |
| 6 | 14-27E-45P | | 50 | 35 | 10 | 10 | 15 | 25 |
| 7 | 14-27E-45P | 3 | 75 | 55 | 35 | 35 | 60 | 175 |
| 8 | 14-27E-55P | | 75 | 60 | 25 | 25 | 30 | 55 |
| 9 | 14-36E-72P | 3 | 75 | >400 | - | - | - | - |
| 10* | 14-52E-58P | 3 | 75 | >400 | - | - | - | - |
| 11* | 14-36E-OP | 3 | 75 | >400 | - | - | - | - |
| 12 | 14-30E-55P | 3 | 75 | 60 | 25 | 10 | 20 | 25 |
| 13 | 14-30E-65P | 3 | 75 | 60 | 30 | 35 | 30 | 25 |

*Not an example of the invention.

## Example 7

The broth of Example 6 was aged for one day at 30°C and retested. The results are in Table VI.

TABLE VI

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | >400 | - | - | - | - |
| 2 | 14-25E-50P | | 50 | 15 | 15 | 25 | 45 | 95 |
| 3 | 14-25E-50P | | 100 | 15 | 10 | 15 | 25 | 55 |
| 4 | 14-25E-50P | | 200 | 15 | 10 | 10 | 10 | 10 |
| 5* | PPG-2000 | | 100 | 40 | 35 | 40 | 45 | 50 |
| 6* | PPG-2000 | | 200 | 35 | 10 | 10 | 10 | 10 |
| 7* | PPG-2000 | | 200 | 30 | 25 | 20 | 25 | 40 |
| 8* | PPG-2000 | | 300 | 35 | 25 | 30 | 30 | 30 |
| 9* | PPG-2000 | | 400 | 10 | 10 | 10 | 10 | 10 |
| 10* | DF-60P[4] | | 100 | 10 | 35 | 85 | 120 | 170 |
| 11* | DF-60P[4] | | 200 | 40 | 35 | 15 | 20 | 30 |
| 12* | DF-60P[4] | | 400 | 0 | 0 | 10 | 35 | 60 |
| 13* | PA-188[5] | | 200 | 200 | 185 | 205 | 210 | 235 |
| 14* | T-701[6] (BASF) | | 200 | 20 | 60 | 160 | 185 | 210 |
| 15* | T-1501[7] (BASF) | | 200 | 225 | 225 | 235 | 235 | 235 |

*Not an example of the invention.

7

## Example 8

In the manner of Example 6, another similar broth having 2% solids, lower viscosity, and a larger particle size was evaluated at 36°C. The results are shown in Table VII.

TABLE VII

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | 135 | 165 | 185 | 220 | 245 |
| 2 | 14-25E-50P | | 50 | 10 | 10 | 15 | 20 | 25 |
| 3 | 14-30E-50P | 3 | 50 | 15 | 20 | 25 | 35 | 70 |
| 4 | 14-30E-50P | 3 | 75 | 15 | 25 | 25 | 25 | 45 |
| 5 | 14-30E-50P | | 50 | 30 | 35 | 40 | 40 | 50 |
| 6 | 14-27E-55P | 3 | 75 | 15 | 15 | 20 | 25 | 25 |
| 7 | 14-36E-72P | 3 | 75 | 130 | 160 | 185 | 215 | 235 |
| 8 | 14-52E-58P | 3 | 75 | 125 | 140 | 185 | 205 | 240 |
| 9 | 14-35E-50P | 3 | 75 | 30 | 30 | 30 | 35 | 35 |
| 10 | 14-27E-45P | 3 | 75 | 10 | 10 | 10 | 10 | 30 |
| 11* | 14-36E-OP | 3 | 75 | 185 | 206 | 215 | 236 | 250 |
| 12* | PPG-2000 | | 50 | 175 | 175 | 195 | 225 | 295 |
| 13* | DF-60P[5] | | 50 | 65 | 125 | 235 | 245 | 260 |

*Not an example of the invention.

## Example 9

The broth of Example 8 was aged for one day at 36°C and reevaluated. The results are reported in Table VIII.

TABLE VIII

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | >400 | - | - | - | - |
| 2 | 14-25E-50P | | 50 | 10 | 10 | 135 | 160 | 305 |
| 3 | 14-25E-50P | | 100 | 20 | 20 | 20 | 20 | 35 |
| 4 | 14-25E-50P | | 1000 | 10 | 10 | 10 | 10 | 10 |
| 5* | PPG-2000[1] | | 100 | 95 | 175 | 245 | >400 | - |
| 6* | PPG-2000[1] | | 500 | 50 | 95 | 105 | 135 | 175 |
| 7* | PPG-2000[1] | | 1000 | 50 | 70 | 85 | 85 | 95 |
| 8* | PPG-2000[1] | | 1500 | 45 | 55 | 85 | 90 | 115 |
| 9* | PPG-2000[1] | | 2000 | 45 | 60 | 65 | 90 | 125 |
| 10* | DF-60P[4] | | 100 | 70 | 205 | 335 | >400 | - |
| 11* | DF-60P[4] | | 500 | 20 | 30 | 75 | 160 | 245 |
| 12* | DF-60P[4] | | 1000 | 10 | 15 | 25 | 105 | 195 |
| 13* | DF-60P[4] | | 1500 | 10 | 20 | 25 | 40 | 85 |
| 14* | DF-60P[4] | | 2000 | 10 | 10 | 10 | 10 | 10 |
| 15* | PA-188[5] | | 100 | 85 | 105 | 110 | 115 | 145 |
| 16* | L-61 (BASF)[8] | | 100 | 85 | 185 | >400 | - | - |
| 17* | T-701 (BASF)[6] | | 100 | 50 | 165 | 200 | 245 | 300 |
| 18 | 14-27E-55P | | 100 | 10 | 10 | 10 | 25 | 40 |
| 19* | mineral oil and silicon fluid | | 100 | >400 | - | - | - | - |
| 20* | kerosene and silicone fluid | | 100 | >400 | - | - | - | - |
| 21* | silicon emulsion | | 100 | 130 | 145 | 150 | 160 | 190 |
| 22* | TRANS 245[9] | | 100 | 125 | 160 | 295 | 335 | >400 |
| 23* | Kurita 322[10] | | 100 | 235 | 225 | 235 | 235 | 285 |
| 24* | PX-97[11] | | 100 | 165 | 160 | 145 | 145 | 70 |
| 25* | PA-01[11] | | 100 | 55 | 75 | 130 | 160 | 230 |
| 26* | SDF-110L[12] | | 50 | >400 | - | - | - | - |

*Not an example of the invention.

Example 10

In a manner similar to Example 6, a commercial, proprietary broth containing a filamentous bacteria (used to produce an antibiotic compound) which was near the end of the fermentation cycle was evaluated. The broth already contained 200 ppm of SAG 471 (a silicone sold by Union Carbide) and 1400 ppm of UCON LB 625 (a polyglycol sold by Union Carbide). In spite of the presence of these two commercial antifoamers, the "blank" control produced 280 ml of foam after five minutes. The test results are shown in Table IX.

TABLE IX

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | 190 | 225 | 255 | 295 | 280 |
| 2 | 14-25E-50P | | 50 | 95 | 105 | 125 | 140 | 145 |
| 3 | 14-25E-50P | | 100 | 115 | 115 | 140 | 160 | 210 |
| 4 | 14-25E-50P | | 200 | 0 | 10 | 30 | 35 | 75 |
| 5 | 14-25E-50P | | 300 | 0 | 10 | 50 | 75 | 110 |
| 6 | 14-25E-50P | | 500 | 5 | 10 | 50 | 70 | 95 |
| 7* | UCON LB 625 | | 500 | 130 | 130 | 105 | 80 | 60 |
| 8 | 14-27E-50P | 3 | 200 | 20 | 30 | 35 | 45 | 60 |
| 9 | 14-27E-50P | 3 | 500 | 10 | 15 | 20 | 25 | 30 |
| 10 | 14-27E-55P | 3 | 500 | 10 | 10 | 10 | 15 | 20 |
| 11 | 14-36E-72P | 3 | 500 | 150 | 155 | 155 | 100 | 95 |
| 12 | 14-30E-65P | 3 | 500 | 15 | 20 | 25 | 25 | 25 |
| 13 | 14-30E-65P | 3 | 200 | 30 | 30 | 30 | 25 | 30 |

*Not an example of the invention.

## Example 11

A broth similar to that in Example 10, but closer to the beginning of the fermentation cycle and containing no UCON LB 625 was evaluated. These results are shown in Table X.

TABLE X

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | 155 | 205 | 225 | 225 | 205 |
| 2 | 14-25E-50P | | 200 | 0 | 0 | 0 | 0 | 0 |

*Not an example of the invention.

## Example 12

A broth containing a mammalian cell culture for the production of Tissue Plasminogen Activator was evaluated. This broth was known to have a high protein content but no oils present. The results are shown in Table XI.

TABLE XI

| Run | Defoamer | Diluent | Defoamer Dose (ppm) | Foam Height at X min (ml) | | | | |
|---|---|---|---|---|---|---|---|---|
| | | | | 0.5 min | 1 min | 2 min | 3 min | 5 min |
| 1* | None | | 0 | >400 | - | - | - | - |
| 2 | 14-25E-50P | | 200 | 0 | 0 | 5 | 10 | 20 |
| 3 | 14-25E-50P | | 100 | 10 | 15 | 10 | 10 | 10 |
| 4 | 14-25E-50P | | 50 | 10 | 5 | 5 | 0 | 5 |
| 5 | 14-25E-50P | | 25 | 10 | 10 | 5 | 5 | 0 |
| 6 | 14-25E-50P | | 10 | 185 | 175 | 160 | 145 | 135 |
| 7 | 14-30E-65P | | 200 | 15 | 35 | 25 | 10 | 15 |
| 8 | 14-30E-65P | | 1000 | 185 | 85 | 125 | 185 | 215 |

*Not an example of the invention.

Example 13

The broth of Example 12 was aerated to produce 400 ml foam. 25 ppm of 14-25E-50P was dropped on the foam, causing it to completely disappear in less than 3 seconds.

Example 14

Several more broths similar to those used in Examples 1-3 were evaluated with similar results obtained.

Comparative Example 1

Several liquids from the commercial production of pork insulin were evaluated. These liquids were not fermentation broths. The compounds of the invention performed poorly, while several silicone products exhibited good performance.

Example 15

A commercial fermentation broth used for antibiotic production was evaluated after heating to 30°C. The broth had not been sterilized or inoculated. The results are shown in Table XII.

TABLE XII

| Run | Defoamer | Diluent | Defoamer Dose (ppm) | Foam Height at X min (ml) | | | | | |
|-----|----------|---------|---------------------|---------|-------|-------|-------|-------|-------|
| | | | | 0.5 min | 1 min | 2 min | 3 min | 4 min | 5 min |
| 1* | None | | 0 | >400 | - | - | - | - | - |
| 2 | 14-10E-40P | | 100 | 35 | 20 | 25 | 35 | 35 | 45 |
| 3 | 14-10E-50P | | 100 | 35 | 25 | 25 | 30 | 35 | 35 |
| 4 | 14-10E-60P | | 100 | 110 | 60 | 25 | 25 | 30 | 35 |
| 5 | 14-15E-40P | | 100 | 30 | 35 | 40 | 45 | 30 | 55 |
| 6 | 14-15E-50P | | 100 | 25 | 20 | 30 | 35 | 35 | 35 |
| 7 | 14-15E-60P | | 100 | 25 | 20 | 25 | 30 | 35 | 40 |
| 8 | 14-17.5E-40P | | 100 | 15 | 25 | 35 | 35 | 35 | 45 |
| 9 | 14-17.5E-50P | | 100 | 25 | 30 | 35 | 40 | 40 | 40 |
| 10 | 14-17.5E-60P | | 100 | 15 | 20 | 25 | 30 | 30 | 35 |
| 11 | 14-20E-20P | | 100 | 50 | 60 | 130 | 225 | >400 | - |
| 12 | 14-20E-30P | | 100 | 20 | 20 | 20 | 20 | 15 | 15 |
| 13 | 14-20E-40P | | 100 | 20 | 15 | 15 | 15 | 15 | 15 |
| 14 | 14-20E-50P | | 100 | 5 | 10 | 10 | 10 | 10 | 10 |
| 15 | 14-20E-50P | | 100 | 10 | 15 | 15 | 15 | 15 | 15 |
| 16 | 14-20E-60P | | 100 | 10 | 10 | 15 | 20 | 20 | 15 |
| 17 | 14-25E-40P | | 100 | 35 | 35 | 35 | 50 | 60 | 65 |
| 18 | 14-25E-50P | | 100 | 45 | 35 | 35 | 35 | 35 | 35 |
| 19 | 14-25E-50P | | 100 | 20 | 25 | 35 | 40 | 45 | 50 |
| 20 | 14-25E-60P | | 100 | 35 | 30 | 35 | 40 | 45 | 45 |
| 21 | 14-30E-40P | | 100 | 60 | 60 | 55 | 60 | 65 | 75 |
| 22 | 14-30E-50P | | 100 | >400 | - | - | - | - | - |
| 23 | 14-30E-60P | | 100 | >400 | 240 | 250 | 260 | 280 | 280 |

*Not an example of the invention.

Example 16

A broth similar to that of Example 15 was evaluated at 36°C. The results are shown in Table XIII.

TABLE XIII

| Run | Defoamer | Diluent | Defoamer Dose (ppm) | Foam Height at X min (ml) | | | | | |
|-----|----------|---------|---------------------|---------|-------|-------|-------|-------|-------|
| | | | | 0.5 min | 1 min | 2 min | 3 min | 4 min | 5 min |
| 1* | None | | 0 | >400 | - | - | - | - | - |
| 2 | 14-10E-40P | | 100 | 25 | 30 | 40 | 45 | 60 | 60 |
| 3 | 14-10E-50P | | 100 | 20 | 20 | 35 | 45 | 50 | 60 |
| 4 | 14-10E-60P | | 100 | 20 | 25 | 30 | 45 | 55 | 60 |
| 5 | 14-15E-40P | | 100 | 20 | 30 | 45 | 70 | 115 | 260 |
| 6 | 14-15E-50P | | 100 | 15 | 20 | 30 | 45 | 55 | 60 |
| 7 | 14-15E-60P | | 100 | 20 | 20 | 25 | 45 | 60 | 85 |
| 8 | 14-17.5E-40P | | 100 | 10 | 20 | 40 | 60 | 160 | 210 |
| 9 | 14-17.5E-50P | | 100 | 25 | 30 | 50 | 60 | 110 | 245 |
| 10 | 14-17.5E-60P | | 100 | 10 | 10 | 20 | 35 | 40 | 50 |
| 11 | 14-20E-20P | | 100 | 35 | 85 | >400 | - | - | - |
| 12 | 14-20E-30P | | 100 | 25 | 30 | 30 | 35 | 60 | 85 |
| 13 | 14-20E-40P | | 100 | 25 | 35 | 45 | 55 | 50 | 45 |
| 14 | 14-20E-50P | | 100 | 25 | 25 | 30 | 35 | 35 | 40 |
| 16 | 14-20E-60P | | 100 | 25 | 25 | 25 | 30 | 35 | 35 |
| 17 | 14-25E-40P | | 100 | 40 | 50 | 70 | 235 | 360 | 400 |
| 18 | 14-25E-50P | | 100 | 45 | 45 | 45 | 45 | 55 | 140 |
| 20 | 14-25E-60P | | 100 | 25 | 35 | 40 | 35 | 40 | 45 |
| 21 | 14-30E-40P | | 100 | 100 | 110 | 115 | 115 | 125 | 180 |
| 22 | 14-30E-50P | | 100 | 160 | 160 | 120 | 125 | 185 | 235 |
| 23 | 14-30E-50P | | 100 | 35 | 35 | 50 | 65 | 110 | 210 |
| 24 | 14-30E-50P | | 100 | 70 | 85 | 75 | 70 | 45 | 50 |

*Not an example of the invention.

Example 17

Another broth similar to that of Example 15 was evaluated at 36°C. The results are shown in Table XIV.

TABLE XIII

| Run | Defoamer | Diluent | Defoamer Dose (ppm) | Foam Height at X min (ml) | | | | | | |
|-----|----------|---------|---------------------|---------|-------|-------|-------|-------|-------| |
| | | | | 0.5 min | 1 min | 2 min | 3 min | 4 min | 5 min | |
| 1* | None | | 0 | >400 | - | - | - | - | - | |
| 2 | 14-20E-40P | | 100 | 15 | 20 | 25 | 25 | 25 | 25 | |
| 3 | 14-20E-50P | | 100 | 20 | 15 | 20 | 20 | 20 | 20 | |
| 4 | 14-20E-60P | | 100 | 25 | 25 | 20 | 15 | 20 | 20 | |
| 5 | 14-20E-60P | | 30 | 25 | 25 | 35 | 55 | 70 | 95 | |
| 6 | 14-10E-40P | | 100 | 35 | 35 | 45 | 50 | 60 | 80 | |

*Not an example of the invention.

1. A polypropylene glycol having a molecular weight of 2000.

2. 50% active ingredient and 50% methanol.

3. 66% solid product, 20% methanol, and 14% water.

4. A polyalkylene glycol reacted with propylene oxide and ethylene oxide sold by Mazer Chemical.

5. A hydrophobized silica in paraffin oil sold by U.S. Movidyn.

6. A polyethoxylated polypropoxylated ethylene diamine (molecular weight = 3600) sold by BASF.

7. A polyethoxylated polypropoxylated ethylene diamine (molecular weight = 7900) sold by BASF.

8. A propoxylated ethoxylated dipropylene glycol (molecular weight = 2000) sold by BASF.

9. A composition of unknown chemistry sold by Trans-Chemco, Inc.

10. A ester of oleic acid and polyoxyalkylene glycol (molecular weight = 2000-3000) sold by Kurita Water Co.

11. A composition of unknown chemistry sold by U.S. Movidyn Co.

12. An acetylenic diol compound made by Air Products.

## Claims

1. A method of inhibiting the formation of foam or reducing existing foam in a fermentation broth comprising adding to the broth an effective amount of a compound of the formula

$RO(EtO)_n(PrO)_mH$

wherein R is a $C_4$ to $C_{30}$ hydrocarbon radical, EtO is an ethoxy moiety, PrO is a propoxy moiety, n is from 10 to 40, and m is from 20 to 80.

2. The method of claim 1 wherein R is an unsubstituted n-alkyl moiety.

3. The method of claim 2 wherein R has 8 to 18 carbon atoms.

4. The method of claim 3 wherein R has 12 to 16 carbon atoms.

5. The method of any of claims 2 to 4 wherein n is from 10 to 30.

6. The method of claim 5 wherein n is from 15 to 25.

7. The method of any of claims 2 to 6 wherein m is from 30 to 50.

8. The method of claim 7 wherein m is from 35 to 45.

9. The method of any preceding claim wherein the fermentation broth is for the production of a pharmaceutical.